# EUROPEAN PATENT SPECIFICATION

(11) **EP 0 980 363 B1**
(45) Date of publication and mention of the grant of the patent: **10.03.2010**
(21) Application number: 98916661.6
(22) Date of filing: 28.04.1998
(51) Int. Cl.: C12P 17/06, A61K 31/35

(54) **PREPARATION OF ISOFLAVONES FROM LEGUMES**
HERSTELLUNG VON ISOFLAVONEN AUS LEGUMINOSEN
PREPARATION D'ISOFLAVONES À PARTIR DE LÉGUMINEUSES

(30) Priority: 28.04.1997 AU PO644897
(43) Date of publication of application: 23.02.2000
(73) Proprietor: Novogen Inc., Wilmington, DE 19805 (US)
(72) Inventor: KELLY, Graham, Edmund, Northbridge, NSW 2063 (AU); HUANG, Jiu, Li, Lane Cove, NSW 2066 (AU); DEACON-SHAW, Mark, G., Koolewong, NSW 2256 (AU); WARING, Mark, A., Elanora Heights, NSW 2010 (AU)
(74) Representative: Goulard, Sophie
(86) International application number: PCT/AU1998/000305
(87) International publication number: WO 1998/049153

(56) References cited:
- AU-A- 7 800 294
- AU-A- 7 839 994
- HESSLER ET AL: "Isolation of isoflavones from soy-based fermentations of the erythromycin-producing bacterium Saccharopolyspora erythraea" APPLIED MICROBIOLOGY AND BIOTECHNOLOGY, vol. 47, April 1997 (1997-04), pages 398-404, XP002498842
- DERWENT ABSTRACT, Accession No. 95-272884/36, Class B02; & JP,A,07 173 148 (KIKKOMAN CORP) 11 July 1995.

## Description

Isoflavones are plant chemicals which occur largely in members of the *Leguminosae* plant family. They are based on a simple diphenolic ring structure as described for example by Carlson et al (1980) Journal of Chromotography, 198, 193-197.

Over 700 different isoflavones are described and these display a range of biological functions both within the plant and within animals including humans which eat the isoflavone-containing plants.

A small sub-group of isoflavones (daidzein, genistein, biochanin, formononetin and glycitein) are distinguished by their ability to bind to estrogen receptors on animal (including human) cells. This is due to the close similarity of the steric structure of the diphenolic rings of isoflavones with the steroidal ring structure of estrogens such as estradiol, estrone.and estriol. Although having substantially lower binding affinity to the receptor compared to steroidal estrogens, estrogenic isoflavones are weakly estrogenic. This group also exhibits a range of biological functions in animal cells which appear to be independent of the estrogen receptor and these include anti-oxidant, diuretic, anti-spasmolytic and anti-cancer effects. These interesting functions with their potential therapeutic benefits has brought this particular group of isoflavones to the attention of medical researchers in recent years.

In the plant, the isoflavones can occur in a variety of forms - (i) in the basic aglucone form, (ii) as a-glucone, being bound to a sugar moiety such as glucose via a β-glucosidic linkage (the glycosidec form), (iii) the glucone form + a malonyl moiety, and (iv) the glucone form + an acetyl moiety as described for example, by Carlson et al (1980) as referred to above.

The glycosidic form (either alone or in the malonyl or acetyl forms) is water-soluble and is the predominant form for the isoflavones in many legumes to facilitate transport and storage. The glycosidic form provides enhanced stability to degradative factors such as heat, oxidation and ultraviolet irradiation. At the intra-cellular site of biochemical function of the isoflavone, an intra-cellular β-glycosidase enzyme cleaves the sugar moiety where present, leaving the more biologically active, but substantially water-insoluble, aglucone form.

Isoflavones are fairly widely distributed within the plant kingdom although they are found predominantly in members of the *Leguminosae* family. The estrogenic isoflavones (genistein, biochanin, formononetin, daidzein, glycitein) follow this general rule in being largely restricted to the genus *Leguminosae.* Most legumes investigated have been found to contain at least detectable levels of one or more of these five estrogenic isoflavones but the richest sources are the legumes.- soya, lentils, chick peas, fenugreek, clovers, alfalfa and various varieties of beans. The richest sources of these compounds are the clovers (including *Trifolium pratense, Trifolium sub-terranean*) and soya (either whole soya or defatted soya or any materials ensuing as products of soya processing including soya grits, soya hypocotyls and soy molasses). The levels of these compounds in clovers and soya varies according to the variety or cultivar and on seasonal, environmental and plant age factors. Levels in clovers vary between about 0.5 and 3.5% (on dry weight basis) and in soybeans between about 0.05 and 0.3% (dry weight).

Isoflavones may be used as therapeutics for pre-menstrual syndrome and menopausal syndrome (US Patents 5569459, 5516528, 5498631) and osteoporosis (US Patent 5424331) and as food additives (US Patents 4366082, 4390559). Given these important uses, it is advantageous to isolate or to concentrate isoflavones from plants.

Various techniques have been proposed to achieve isolation of isoflavones, but essentially there are two distinct methods. The first method involves the conversion of the water-soluble glucone form to the water-insoluble aglucone form to facilitate the subsequent extraction of the aglucones in a suitable organic solvent such as alcohol. This conversion step is described as being achieved in one of two ways: either (a) through hydrolysis by exposure to vigorous heating (typically 80-100°C) at low pH (Wang K, Kuan SS, Francis OJ, Ware KM, Carman AS. "A simplified HPLC method for the determination of phytoestrogens in soybean and its processed products. " J. Agric. Food Chem. 38:185-190, 1990)*;* or (b) by exposure to an enzyme (glucose hydrolase, β-glycosidase or β-glucuronidase) which specifically cleaves the β-glycosidic linkage with the sugar moiety. The enzyme either is added to the reaction or the naturally occurring β-glucosidase within the plant can be utilised. In respect to the latter, a method is described (JP 89-345164/47) whereby the natural enzyme activity within soybeans is utilised by heating soyflour to 45-55°C for several hours, although the amount of naturally-occurring enzyme activity in commercially available soyflour samples is highly variable and even when at its maximum is insufficient to obtain hydrolysis of more than about 50-60% of the glucones present.

The various hydrolysis reaction procedure (either enzymatic or heating/low pH) are described as being applied to an admixture of ground plant material in water. At the conclusion of the hydrolysis process, the aqueous phase is separated from the undissolved plant material to facilitate the next step. Once the conversion of the glucone to the aglucone form is achieved, the aqueous mixture then is contacted with an organic (and water immiscible) solvent. The aglucones due to their substantial water insolubility are extracted into the organic solvent phase and subsequently recovered.

Previously proposed methods involve initial water extraction of the isoflavones in their glycosidic form so that they either are retained in this form or can be converted subsequently to their aglucone form. Techniques described for this approach involve adding the ground plant material to water and over a period of time (several hours to several days) the naturally-occurring glycosidic forms of the isoflavones dissolve in the aqueous phase. After separating the undissolved plant material from the aqueous phase, the isoflavones in the aqueous phase are converted to the aglucone form by any of the methods outlined above and subsequently recovered. A variant of this approach involves selective removal of the aglucone forms from the aqueous mixture by absorption on to an appropriate ion-exchange resin. The isoflavones subsequently are eluted from that resin using a water:organic solvent mixture, concentrated by rotary evaporation, and then hydrolysed to the aglucone form by enzymatic digestion or heat/acid treatment (JP 95-272884/36).

Disadvantages of the above techniques include (a) a multiplicity of steps, (b) the use of vigorous treatments such as heating and/or strong acid and/or strong alkali, (c) the comparatively low yields of isoflavones, (d) the very high cost of hydrolysing enzymes, and (e) the high capital costs and high running costs associated, with large-scale multiple step extraction of isoflavones in commercial quantities. All of the current known isoflavone extraction procedures are affected by one or more of there disadvantages and serve to greatly reduce the commercial viability of the process. If the purported community health benefits of the estrogenic isoflavoes are to be realised then they must become economically accessible to the general community. For this to happen, an improved and more cost-effective method of extraction must be found.

### Summary of the Invention

In the broadest aspect of this invention there is provided processes for the production of isoflavones from plants of the genus *Leguminosae* which comprises contacting plant material with water, a C₁-C₁₀ organic solvent and an enzyme which cleaves isoflavone glycosides to the aglucone form, to form a combination, and incubating the combination for a time sufficient to allow isoflavones of the aglucone form to partition into the organic solvent, and thereafter recovering isoflavones from the organic solvent.

The combination of the aforementioned components may comprise an aqueous phase containing enzyme and plant material and an organic phase into which the isoflavones partition. The combination may alternatively comprise an emulsion formed: by vigorous mixing of the organic solvent and water, or if a .water miscible organic solvent is used the combination is a mixture of water and organic solvent.

Where the organic solvent is non-water miscible the organic solvent containing dissolved isoflavones may be removed, for example by evaporation to give an isoflavone containing residue. The residue may then be mixed with a C₁-C₁₀ organic solvent in which isoflavones are substantially insoluble such that isoflavones precipitate and are subsequently recovered.

Where the organic solvent is miscible with water, the organic solvent, in the combination may be removed, for example by evaporation, to give an isoflavone containing residue and water which may be thereafter mixed with a non-water miscible C₁-C₁₀ isoflavone solubilising organic solvent to give an organic and an aqueous phase. The organic solvent phase containing dissolved isoflavones may be collected and isoflavones recovered therefrom. The organic solvent may be evaporated with water addition whereafter isoflavones form a water insoluble flocculate which is subsequently recovered.

An enzyme used to cleave isoflavone glycosides to the aglucone form preferably includes a β glucanase. More preferably the enzyme is a mix (or combination) of β-glucanase and β-xylanase.

### Detailed Description of the Invention

The present invention provides in its broadest aspect a process for the production of isoflavones from plants of the genus *Leguminosae* which comprises contacting plant material with water, a C₁-C₁₀ 10 organic solvent and an enzyme which cleaves isoflavone glycosides to the aglucone form, to form a combinations and incubating the combination for a time sufficient to allow isoflavones of the aglucones form to partition into the organic solvent, and thereafter recovering isoflavones from the organic solvent.

The combination which results from combining together the plant material, water, a C₁-C₁₀ organic solvent and an enzyme which cleaves isoflavone glycosides to the aglucone form, may be in the form of a separated phase mixture comprising an aqueous phase containing the enzyme and plant material and an organic phase into which isoflavones partition on incubation following cleavage by the enzymes. The combination may comprise an emulsion formed by vigorous mixing of the organic solvent and water, or where the organic solvent is water miscible the combination may be a mixture of water and organic solvent. Where the combination comprises an emulsion it is preferred to remove particulate material from the emulsion, after a period of time sufficient to enable the aglucones form of the isoflavone to partition into the organic solvent, using a standard separation process such as filtration or centrifugation. Phase separation then occurs, this subsequently allowing recovery of isoflavones from the organic solvent component.

Where the organic solvent is non-water miscible the organic solvent component containing dissolved isoflavones may be removed, for example by evaporation to give an isoflavone containing residue. The residue may then be mixed with a C₁-C₁₀ organic solvent in which isoflavones are substantially insoluble such that isoflavones precipitate and are subsequently recovered,

Where the organic solvent is miscible with water, the organic solvent in the combination may be removed, for example, by evaporation, to give an isoflavone containing residue and water which may be thereafter mixed with a non-water miscible C₁-C₁₀ isoflavone solubilising organic solvent to give an organic and an aqueous phase, The organic solvent phase containing dissolved isoflavones may be collected and isoflavones recovered therefrom. The organic solvent may be evaporated with water addition whereafter isoflavones form a water insoluble flocculate which is subsequently recovered.

The enzyme used to cleave the isoflavone glycoside to the aglucone form (hereinafter referred to as isoflavone) is required specifically to cleave the β-glycosidic linkage which is described as the dominant linkage between the isoflavone and its carbohydrate (normally glucose) moiety. A person skilled in the field of carbohydrate chemistry would deduce that the most appropriate enzyme to achieve this would be a β-glucosidase and possibly a β-glucanase. As Table 1 shows, in an experiment to compare the relative potencies of different carbohydrate enzymes in their ability to cleave the glycosidic linkage of soy isoflavones, it was found that β-glucosidase was highly effective; β-glucuronidase was found unexpectedly also to be highly effective; β-glucanase unexpectedly was found to have relatively low potency and required a considerably longer reaction time. In some isoflavone containing plants such as clovers, endogenous β-glycosidase enzyme activity is generally sufficient to effect cleavage of the glucone form without the need for additional cleavage enzymes. Hence, enzyme addition may, in the process of this invention, be regarded as optional.

**Table 1**

| Comparative actions of different carbohydrate-acting enzymes in converting soya isoflavones in their glycosidic forms (daidzin, genistin) to the aglucone forms (daidzein, genistein). | |
|---|---|
| **Enzyme type*** | **Relative activity (% conversion)** |
| β-glucosidase | 90 |
| β-glucuronidase | 98 |
| β-glucanase | 40 |
| 1,4-bD-glucan hydrolase | 0 |
| 1,4-a-D-glucan hydrolase | 0 |
| β-xylanase:b-glucanase (10:1) | 85 |
| β-xylanase:b-glucanase (1:1) | 87 |

| | |
|---|---|
| * All enzymes added at the same concentration to a standard amount of isoflavone. | |

A β-glucanase/β-xylanase enzyme mix was found by the inventors to be relatively effective in cleaving the isoflavone glycoside to the aglucone form. This was entirely unexpected given that there was no reason to expect that a β-xylanase would have any effect on the described form of glycosidic linkage on the isoflavone glucone form. Advantageously, this fungal-derived enzyme mix is available in large commercial quantities at an advantageous cost. Although only slightly less efficient than the more specific b-glucosidase and β-glucuronidase enzymes, the latter enzymes are not available in bulk, commercial quantities or at cost-effective prices. Moreover, the low cost of the commercial β-glucanase/β-xylanase enzyme mix allowed the dosage per unit of isoflavone to be increased which more than compensates for the slightly lowered efficiency. In one embodiment of the process involving an enzyme, the organic solvent does not cause significant inactivation of the enzyme used.

The plant material is derived from plants of the genus *Leguminosae* and may be obtained from plants such as soy, clover (including subterranean clover, red clover, and other isoflavone-containing clovers), chickpeas, lentils, beans (such as broad, haricot, kidney, lima and navy beans) which generally contain higher levels of isoflavones than other plants of the genus *Leguminosae.* It is preferred that the plant material be derived from soy or clover although this is not to say that other isoflavone containing plants of the genus *Leguminosae* may not be used in the process of this invention. Where isoflavones are extracted from clovers, the use of an enzyme which cleaves isoflavone glycosides is unnecessary.

The plant material is preferably in fine particulate form, such as a flour produced by grinding or otherwise processing plant material such as clover, soy beans, other beans, chickpeas and lentils. The preferred plant material is soya (*Glycine max*) or clover, such as red clover. Without limiting the present invention, it is preferable to remove as much as possible of parts of the plant that do not contain isoflavones to any great extent in order to reduce the bulk of material to be exposed to the extraction process. For example, about 90% of the isoflavones contained in harvested clovers occurs in the leaves and about 10% in the stalks and petioles so it is advantageous to separate the leaves from the stalks which can be achieved by first exposing the dried plant to a threshing action followed by differential sieving to separate the smaller leaves from the larger stalks. In another example, soybeans may be dehulled and/or defatted and dehulled. Defatted soyflour is readily available in commercial quantities. In another example, soy hypocotyl which often breaks away from the soy cotyledons during regular dehulling processes and is readily collected by standard methods such as sieving, contains typically higher isoflavone levels (between about 0.5 and 1.5%) compared to the whole soybean (between about 0.05 and 0.3%).

The organic solvents utilised in the various embodiments of this invention comprise from 1 to 10 carbons (C₁-C₁₀) and include water immiscible ans water miscible organic solvent. Water-mistible organic solvents include C₁-C₁₀ alcohols such as methanol, ethanol, propanol and isopropanol, acetic acid, acetone, acetonitrile, dimethyl formamide, dimethyl sulphoxide, n-propanol, isopropanol, tetrahydrofuran and mixtures of any such solvents. Water-immiscible C₁-C₁₀ solvents which are isoflavone solubilising include C₄-C₁₀ alcohols (such as butanol, hexanol and pentanol), C₁-C₁₀ alkoxy solvents (such as ethyl methyl ketone, methyl phenyl ketone, hexane-2,4-dione and the like); C₂-C₁₀ esterified acids (such as ethyl acetate, ethyl methyl malonate, dimethyl phosphonate); C₁-C₈ aldehydes (including butanone, pentanone, hexanedial, cyclohexane carbaldehyde and butane-1,2,4-tricarbaldehyde); C₂-C₁₀ ethers, C₂-C₃ alkenes, C₂-C₄ alkanes or phenol and its derivatives (such as benzene 1,2,4-thiol) and mixtures of any such solvents. Organic solvents in which isoflavones are substantially insoluble include C₅-C₁₀ alkanes (such as hexane, cyclohexane, heptane and octane) and C₄-C₁₀ alkenes and mixtures of any such solvents. The organic solvent utilised is preferably selected to have a volatility to enable the organic solvent to be removed by evaporation (for example by distillation, rotary evaporation and the like) so that isoflavone compounds dissolved in the organic solvent can be subsequently recovered.

The water used in the process may be from any conventional water source, distilled water, deionized and distilled water or the like. The water may contain preservatives to retard microbial growth and/or other additives as are well known in the art. The respective proportions of water and organic solvent are not limiting on this invention. Generally equal proportions of water and organic solvent are used, although the ratio of water to organic solvent may vary, for example from 1:10 to 10:1.

Where the combination resulting from the mixture of the water and organic solvent comprises an organic phase and an aqueous phase the respective phases may be gently mixed or agitated. This can easily be achieved by a vertically disposed stirrer which allows mixing of the respective phases without intermixing of the phases as such.

The process of the invention does not require elevated temperatures and may be conducted under ambient temperature conditions, for example from 5°C to 35°C. Ambient temperature conditions can therefore suffice without the need for sophisticated temperature control as is required in prior art processes where elevated extraction temperatures are necessary.

In one embodiment the extraction process of the present invention is a one pot, single stage ' reaction which confers substantial benefits such as cost savings in capital equipment expenditure and in time. The efficiency of enzymatic digestion and solvent extraction in one step, according to one embodiment of this invention, is very efficient and gives high yield of isoflavone products, which is generally in contrast to prior art procedures.

Isoflavone compounds are recovered from the organic solvent component generally by vaporisation (evaporation) of the organic phase such as by rotary evaporation; distillation or the like. A small amount of oil containing the aglucone isoflavones is found to remain following removal of the organic phase. This isoflavone-enriched oil may be regarded as the desired end-product and used as such, although it is preferable to continue the extraction process to further concentrate the isoflavones. The oil containing isoflavones may be then removed by the addition of a suitable organic solvent such as hexane, heptane and octane which are highly soluble for oils but very low solubility for isoflavones; hexane preferably is used because of its relatively low cost. The solvent (such as hexane) is added at a ratio to the oil of between about 1:1, and 50:1, preferably 10:1. It is found that the oil readily partitions into the organic solvent phase and that this is associated with the isoflavones falling out of solution and settling to the bottom of the vessel. The hexane:oil phase then is removed leaving the isoflavone-containing residue. This may be recovered and dried, such as in an oven at a temperature between about 50°C to 120°C, to produce a fine powder which is subsequently formulated for therapeutic use as described hereafter. Preferably, however, the hexane extraction step is repeated a further 1-3, times to effect complete removal of oil. Alternatively, the isoflavone containing oil may be subject to HPLC fractionation, ion exchange, chromatography or other techniques well known in the art for isoflavone enrichment/purification.

Where the C₁₋C₁₀ organic solvent used to extract plant material is miscible with water (for example an alcohol such as ethanol), the organic solvent may be removed by evaporation (such as rotary evaporation or distillation) to give a concentrate containing an isoflavone containing oil in water. This concentrate may be mixed with a C₁-C₁₀ isoflavone solubilising organic solvent, for example ethyl acetate to give an organic isoflavone containing phase and an aqueous phase. The organic phase may be Collected and isoflavones recovered therefrom. For example, organic solvent may be evaporated with water addition, for example using a still, whereafter isoflavones form a water insoluble flocculate which is subsequently recovered and formulated into a pharmaceutical/health composition.

At this stage the extracted material is of high isoflavone content, such as from 36 to 70% isoflavones, and generally is comparable to the ratio of isoflavones of the starting material. As a consequence the yields are typically very high. The material may be used for therapeutic purposes at that point, for example being dried and subsequently formulated, or can be subject to further processing as is known in the art to further purify the isoflavone. Further purification may comprise.HPLC fractionation, ion exchange chromatography or other techniques as are well known in the art. For example, by PLC fractionation daidzein or genistein may be removed.

Were soya is the starting material, the isoflavones extracted are daidzein, genistein, and glycitein; the remaining material is composed of a range of compounds including phytosterols and other water-insoluble compounds. Where clover is the starting material, the isoflavones extracted are daidzein, genistein, formononetin and biochanin; various flavonoids including chlorophyll as well as phytosterols make up the bulk of the remainder of the isolate.

The isoflavones produced according to the process of this invention may be.individually purified. For example daidzein and genistein may be purified to HPLC, or other chromatographic techniques or standard methods for purifying these compounds known in the art.

The isoflavones may be formed into pharmaceutical compositions or health compositions, drinks, foods and the like, in combination with appropriate excipients, carrier and the like as are well known in the art, for example as described in Handbook of Pharmaceutical Excipients, Second Edition, American Pharmaceutical Association, 1994. Pharmaceutical compositions or health compositions may comprise tablets, capsules, powders for reconstitution, syrups and the like. Standard carriers/excipients used in such formulations include microcrystalline cellulose, calcium hydrogen phosphate, magnesium stearate and colloidal silica. Foods containing isoflavones may comprise food bars, biscuits, snack foods and other standard food forms well known in the art. Drinks may contain flavouring, buffers and the like.

It would appear that the prior art has not contemplated the use of a one pot process for conversing isoflavones from the aglucone to the aglucone form at the same time as recovery of the aglucone isoflavones in an organic solvent for a number of reasons. It may have been believed necessary to remove residual leguminous plant material from the process after cleavage of the glycoside form. It may also have been regarded that organic solvent would inactivate enzymes which effect formation of the aglucone form. As a consequence, in the prior art conversion of the water soluble glucone form to the water insoluble aglucone form has been carried out in multiple steps, followed by a subsequent step of extraction of the aglucones in a suitable organic solvent.

Embodiments of the present invention will now be described with reference to the following non-limiting examples.

### Example 1

2000 kg of defatted soyflour is placed in a 10,000 L vessel as depicted in Figure 1 containing 5,000 L of deionised water and 10 kg of β-glucanase/b-xylanase (Bio-Feed Beta CT; Novo Nordisk, Denmark), 1000 L of ethyl acetate is then layered on top of the aqueous suspension to give a two phase combination. Both aqueous and solvent phases are gently mixed by continuous stirring using a vertical propeller mixer (Figure 1). It is found that at the point of contact between the aqueous and organic solvent phases, the aglucone isoflavones readily move from the aqueous to the organic solvent phase. The constant agitation of the aqueous phase is designed to ensure maximum exposure of the hydrolysed isoflavones to the ethyl acetate; the constant agitation of the ethyl acetate helps to ensure a high isoflavone concentration gradient between the two phases, thereby maximising the rate of dissolution of the water-insoluble aglucone form into the ethyl acetate. An optional further contact between the two phases may be provided by circulating the lower aqueous suspension through the ethyl acetate phase.

After about 4 to about 48 hours, but preferably around 18 hours, the agitation and recirculation processes are stopped and the two phases allowed to separate maximally. The ethyl acetate is removed and evaporated using a still. About 20 L of oil remains unevaporated. 200 L of hexane is added to the oil and mixed vigorously by stirring for about 5 minutes. This is allowed to stand overnight (about 18 hours) without stirring and it is found that particulate material containing the aglucone isoflavones settles to the bottom of the reaction vessel. The hexane:oil phase is decanted leaving a sludge. A further 5 1 of hexane is added to the sludge to effect removal of residual oil. This mixture is allowed to stand for 1 hour by which time the particulate material has settled out once again. The hexane:oil phase is decanted leaving a semi-solid sludge which is collected and dried in an oven at a temperature of about 85°C: By HPLC analysis this material is found to contain between about 36-70% (typically about 60%) isoflavones. Importantly, the ratio of the isoflavones in the extract is comparable to that of the starting material and the isoflavone yields typically are very high (Table 2). This material can be used for the purpose as is, or can be subjected to further processing in order to further purify the isoflavone.

**Table 2**

| Recovery of isoflavones from whole soyflour using the extraction method described in Example 1. | |
|---|---|
| Isoflavone | % recovery of starting material |
| daidzein | 80:3 |
| genisten | 76.3 |
| glycitein | 75.0 |

### Example 2

The starting material is 200 kg of soy, grits containing a mixture of soy hypocotyls and pieces of soy cotyledons and representing a more enriched source of isoflavones (about 10. % compared to about 0.2% on whole soya flour). 200 kg of soy grits is placed in a 3000 L vessel containing 1000 L of deionised water and 2.5 kg of glucan hydrolase (Bio-Feed Beta CT; Novo Nordisk, Denmark). 1000 L of ethyl acetate is then added and the aqueous and solvent phases then mixed together vigorously using a pump with a capacity of about 200 L per minute to ensure effective contact between the two phases, that is, form an emulsion. The mixing continues at room temperature for a period of between 1-24 hours, but preferably 4 hours. The particulate material in this combination is then separated from the liquid phase by a standard process such as filtration or centrifugation. The removal of the particulate material destroys the emulsion, and on allowing the resulting liquid phase to stand for about 30 minutes there is effected separation between the aqueous and the ethyl acetate phases. The ethyl acetate which contains the isoflavones then is removed and exposed to distillation. The residual oil remaining after distillation of the ethyl acetate then is treated according to the steps outlined in Example 1 above to isolate the isoflavone-enriched material.

### Example 3

550 kg of clover is fed into a counter-current extraction unit and mixed with 5000 L of 50% ethanol for a period of 6 hours. The solvent extract is then pumped to storage and the clover discarded. The ethanol is then recovered by rotary evaporation under pressure (-80kPa) and at 80°C resulting in 500 L of extract concentrate (an isoflavone containing oil in water) and recovery of 4000 L of an ethanol/water mixture. The concentrate is mixed with ethyl acetate at a ratio of 1:4 (i.e. 2000 L ethyl acetate) and the mixture left to settle into a water layer and an ethyl acetate layer. The isoflavones are solubilised into the ethyl acetate layer. The ethyl acetate layer is pumped into a stilt, and the solvent evaporated under vacuum with water addition. The wet floc (active component) is then pumped to a storage tank. 50% of the wet floc is then mixed with a spray drying agent, spray dried and active isoflavones recovered (25%). The remaining 50% is washed with hexane, dewatered, dried at 90°C, milled and mixed with carriers/excipients for tableting.

### Example 4

The dried end product of Examples 1 to 3 above (Sample 1) can be used as starting material to concentrate genistein or daidzein with/without glycitein. Purification is established by standard procedures including HPLC, - ion exchange chromatography and other chromatographic separation. In one series of experiments, 3 kg of the dried end product of Examples 1 to 3 is fractionated allowing separation of daidzein and genistein. Daidzein, of purity between about 95-99% (typically 98.5% purity) is isolated. Genistein of similar purity is recovered.

### Example 5

Pharmaceutical compositions can be prepared from the products extracted according to the examples above.
1. The following composition is prepared in the form of a tablet:
   *Using soyflour extract prepared according to Example I and containing (genistein 35% and daidzein 28% by weight*)
   60 mg of extract
   340 mg of a standard tablet inert carrier
   This composition is tableted to provide a 400 mg tablet containing 20 mg of
   genistein and 17 mg of daidzein.
2. The following composition is prepared in the form of a capsule:
   *Using soy hypocotyl extract prepared according to Example 2 and containing*
   *(genistein 18%, daidiein 35 % and glycitein 18% by weight)*
   60 mg of extract
   190 mg of a standard pharmaceutical inert carrier
   All contained in a non-toxic gelatin capsule and providing 200 mg containing approximately 11 mg of genistein, 21 mg of daidzein and 11 mg of glycitein.
3. The following composition is prepared in the form of a tablet:
   *Using a genistein extract prepared according to Example 4 and containing (genistein 99. 5 % by weight*)
   50 mg of extract
   150 mg of a standard tablet inert carrier
   This composition is tableted to provide a 200 mg tablet containing 50 mg of genistein.
4. The following composition is prepared in the form of a tablet:
   *A 500 mg tablet containing 40 mg of isoflavone prepared according to Example 3 and 460 mg inert excipients*/*carriers.*
   The carriers referred to above include cellulose (microcrystalline), calcium hydrogen phosphate, soy polysacchardie, magnesium stearate and silica-colloidal (anhydrous).

Throughout this specification and the claims which follow, unless the context requires otherwise, the word "comprise", or variations such as "comprises" or "comprising", will be understood to imply the inclusion of a stated integer or step or group of integers or steps but not the exclusion of any other integer or step or group of integers or steps.

## Claims

1. A process for the production of isoflavones from plants of the genus *Leguminosae,* the process comprising the steps of:
(i) combining starting material from plants of the genus *Leguminosae* with water, a supplemental enzyme which cleaves isoflavone glycosides to the aglucone form, and a C₁-C₁₀ organic solvent;
(ii) incubating the combination for a time sufficient to allow isoflavones of the aglucone form to partition into the organic solvent component; and
(iii) recovering the aglucone forms of said isoflavones from the organic solvent component.

2. A process according to claim 1 wherein the organic solvent is recovered, then organic solvent removed to give an isoflavone residue, and the residue is mixed with an organic solvent in which isoflavones are substantially insoluble such that isoflavones precipitate and are subsequently recovered.

3. A process according to claim 1 wherein the organic solvent in the combination containing isoflavones dissolved therein is water-miscible and is removed to give an isoflavone containing residue and water, which is thereafter mixed with a non-water miscible C₁-C₁₀ isoflavone solubilising organic solvent to give an organic and an aqueous phase, the organic solvent phase containing dissolved isoflavones collected and isoflavones recovered therefrom.

4. A process according to claim 3 wherein the organic solvent phase is evaporated with water addition whereafter isoflavones form a water in soluble flocculate

5. A process according to claim wherein the combination comprises an aqueous phase containing an enzyme and plant material and an organic phase into which the isoflavones partition.

6. A process according to claim 1 wherein the combination comprises an emulsion formed by vigorous mixing of the organic solvent and water:

7. A process according to claim 5 wherein the enzyme is a β-glucanase and β-xylanase mixture.

8. A process according to claim 1 wherein the plant material is mixed with the water and an enzyme whereafter the organic solvent is added so as to form an organic phase and an aqueous phase, or an emulsion formed by vigorous mixing of the organic solvent and water.

9. A process according to claim 1 wherein the plant material is mixed with water, whereafter an enzymes is added with the organic solvent.

10. A process according to claim 1 wherein the plant material is from soy or clover.

11. A process according to claim 1 which is carried out at from 10°C to 30°C.

12. A process according to claim 1 wherein the plant material is in particulate form.

13. A process according to claim 12 wherein the plant material is selected among soy flour or a variable mixture of soy hypocotyls and soy cotyledons.

14. A process according to claim 1 wherein the plant material is clover.

15. A process according to claim 1 wherein daidzein is purified from the recovered isoflavones.

16. A process according to claim 1 wherein genistein is purified from the recovered isoflavones.

## Patentansprüche

1. Verfahren zur Herstellung von Isoflavonen aus Pflanzen der Gattung *Leguminosae,* wobei das Verfahren die folgenden Schritte umfasst:
(i) Zusammenbringen des Ausgangsmaterials von Pflanzen der Gattung *Leguminosae* mit Wassers, einem ergänzenden Enzym, welches Isoflavonglykoside zur Aglykon-Form spaltet, und einem C₁-C₁₀ organischen Lösungsmittel;
(ii) Inkubieren der Kombination für eine Zeit, ausreichend für die Isoflavone in der Aglykon-Form in die organische Lösungsmittelphase überzugehen; und
(iii) Gewinnen der Aglylcon-Formen der Isoflavone aus der organischen Lösungsmittelphase.

2. Verfahren gemäß Anspruch 1, wobei das organische Lösungsmittel wiedergewonnen, das organische Lösungsmittel entfernt wird, um einen Isoflavon-Rückstand zu erhalten, und der Rückstand mit einem organischen Lösungsmittel gemischt wird, in welchem Isoflavone im Wesentlichen unlöslich sind, so dass Isoflavone präzipitieren und anschließend wiedergewonnen werden.

3. Verfahren gemäß Anspruch 1, wobei das organische Lösungsmittel in der Kombination, welches dann gelöste Isoflavone enthält, mit Wasser mischbar ist und entfernt wird, um einen Isollavon-enthaltenden Rückstand und Wasser zu erhalten, dieser anschließend mit einem nicht mit Wasser mischbaren C₁-C₁₀ Isoflavonsolubilisierenden organischen Lösungsmittel gemischt wird, um eine organische und eine wässrige Phase zu erhalten, wobei die organische Lösungsmittelphase enthaltend gelöste Isoflavone gesammelt und die Isoflavone hieraus wiedergewonnen werden.

4. Verfahren gemäß Anspruch 3, wobei die organische Lösungsmittelphase unter Wasserzugabe verdampft wird, wonach Isoflavone ein wasserunlösliches Flockulat bilden.

5. Verfahren gemäß Anspruch 1, wobei die Kombination eine wässrige Phase, enthaltend ein Enzym und Pflanzenmaterial, und eine organische Phase, in welche die Isoflavone partitionieren, umfasst.

6. Verfahren gemäß Anspruch 1, wobei die Kombination eine Emulsion umfasst, die durch intensives Mischen des organischen Lösungsmittels und Wassers entsteht.

7. Verfahren gemäß Anspruch 5, wobei das Enzym ein Gemisch aus β-Glukanase und β-Xylanase ist.

8. Verfahren gemäß Anspruch 1, wobei das Pflanzemnaterial mit dem Wasser und einem Enzym gemischt wird, wonach das organische Lösungsmittel hinzugegeben wird, so dass sich eine organische Phase und eine wässrige Phase oder eine durch intensives Mischen des organischen Lösungsmittels und Wassers gebildete Emulsion bilden.

9. Verfahren gemäß Anspruch 1, wobei das Pflanzenmaterial mit Wasser gemischt wird und anschließend ein Enzym mit dem organischen Lösungsmittel hinzugegeben wird.

10. Verfahren gemäß Anspruch 1, wobei das Pflanzenmaterial von Soja oder Klee stammt.

11. Verfahren gemäß Anspruch 1, welches bei 10 °C bis 30 °C durchgeführt wird.

12. Verfahren gemäß Anspruch 1, wobei das Pftanzenmaterial in zerkleinerter Form vorliegt.

13. Verfahren gemäß Anspruch 12, wobei das Pflanzenmaterial ausgewählt ist aus Sojamehl oder einem variablen Gemisch von Soja-Hypokotyten und Soja-Kotyledonen.

14. Verfahren gemäß Anspruch 1, wobei das Pflanzenmaterial von Klee stammt.

15. Verfahren gemäß Anspruch 1, wobei Daidzein aus den gewonnenen Isoflavonen gereinigt wird.

16. Verfahren gemäß Anspruch 1, wobei Genistein aus den gewonnenen Isoflavonen gereinigt wird.

## Revendications

1. Procédé de production d'isoflavones à partir de plantes du genre *Leguminosae,* le procédé comprenant les étapes suivantes :
(i) la combinaison d'une matière première provenant de plantes du genre *Leguminosae* avec de l'eau, d'une enzyme supplémentaire qui clive les glycosides d'isoflavone en forme aglycone, et d'un solvant organique en C₁ à C₁₀ ;
(ii) l'incubation de la combinaison pendant une durée suffisante pour permettre aux isoflavones de la forme aglycone de se partager dans le composant solvant organique ; et
(iii) la récupération des formes aglycones desdites isoflavones dans le composant solvant organique.

2. Procédé selon la revendication 1, dans lequel le solvant organique est récupéré, le solvant organique est éliminé pour donner un résidu d'isoflavones, et le résidu est mélangé avec un solvant organique dans lequel les isoflavones sont sensiblement insolubles, de sorte que les isoflavones précipitent et sont ensuite récupérées.

3. Procédé selon la revendication 1, dans lequel le solvant organique de la combinaison dans lequel des isoflavones sont dissoutes est miscible à l'eau et est éliminé pour donner un résidu contenant des isoflavones et de l'eau, lequel est ensuite mélangé avec un solvant organique non miscible à l'eau solubilisant les isoflavones en C₁ à C₁₀ pour donner une phase organique et une phase aqueuse, la phase de solvant organique contenant des isoflavones collectées et des isoflavones récupérées dissoutes par celui-ci.

4. Procédé selon la revendication 3, dans lequel la phase de solvant organique est évaporée en ajoutant de l'eau, après quoi les isoflavones forment un floculat non soluble dans l'eau.

5. Procédé selon la revendication 1, dans lequel la combinaison comprend une phase aqueuse contenant une enzyme et une substance végétale et une phase organique dans laquelle les isoflavones se partagent.

6. Procédé selon la revendication 1, dans lequel la combinaison comprend une émulsion formée par le mélange vigoureux du solvant organique et de l'eau.

7. Procédé selon la revendication 5, dans lequel l'enzyme est un mélange de β-glucanase et de β-xylanase.

8. Procédé selon la revendication 1, dans lequel la substance végétale est mélangée avec l'eau et une enzyme, après quoi le solvant organique est ajouté afin de former une phase organique et une phase aqueuse, ou une émulsion est formée par le mélange vigoureux du solvant organique et de l'eau.

9. Procédé selon la revendication 1, dans lequel la substance végétale est mélangée avec de l'eau, après quoi une enzyme est ajoutée avec le solvant organique.

10. Procédé selon la revendication 1, dans lequel la substance végétale provient du soja ou du trèfle.

11. Procédé selon la revendication 1, qui est mis en oeuvre entre 10 °C et 30 °C.

12. Procédé selon la revendication 1, dans lequel la substance végétale est sous une forme particulaire.

13. Procédé selon la revendication 12, dans lequel la substance végétale est choisie parmi la farine de soja ou un mélange variable d'hypocotyles de soja et de cotylédons de soja.

14. Procédé selon la revendication 1, dans lequel la substance végétale est du trèfle.

15. Procédé selon la revendication 1, dans lequel de la daidzéine est purifiée à partir des isoflavones récupérées.

16. Procédé selon la revendication 1, dans lequel de la génistéine est purifiée à partir des isoflavones récupérées.
